# EUROPEAN PATENT SPECIFICATION

(11) **EP 1 558 265 B1**
(45) Date of publication and mention of the grant of the patent: **06.01.2010**
(21) Application number: 03769738.0
(22) Date of filing: 05.11.2003
(51) Int. Cl.: A61K 31/585, A61P 15/00

(54) **USE OF DROSPIRENONE FOR THE TREATMENT OF HYPERTENSION**
VERWENDUNG VON DROSPIRENON ZUR BEHANDLUNG VON HYPERTENSION
UTILISATION DE LA DROSPIRENONE POUR LE TRAITMENT DE L'HYPERTENSION

(30) Priority: 05.11.2002 WO PCT/IB02/04628; 05.11.2002 US 287780
(43) Date of publication of application: 03.08.2005
(73) Proprietor: Bayer Schering Pharma Aktiengesellschaft, 13353 Berlin (DE)
(72) Inventor: MEYERHOFER, Siegried, 1230 Vienna (AT)
(74) Representative: Plougmann & Vingtoft A/S
(86) International application number: PCT/IB2003/004946
(87) International publication number: WO 2004/041289

(56) References cited:
- WO-A-01/52857
- WO-A-02/09683
- WO-A-02/17895
- WO-A-02/055086
- US-A1- 2002 136 775
- NORMAN P. ET AL: 'Drospirenone' DRUGS OF THE FUTURE vol. 25, no. 12, December 2000, pages 1247 - 1256, XP000993474
- KRATTENMACHER R.: 'Drospirenone: pharmacology and pharmacokinetics of a unique progesterone' CONTRACEPTION vol. 62, July 2000, pages 29 - 38, XP000993492
- BERGER V. ET AL: 'Influence of different progestogens on blood pressure of non-anaesthetized male spontaneously hypertensive rats' CONTRACEPTION vol. 46, 1992, pages 83 - 97
- WHITE W. ET AL: 'Effects of a new hormone therapy, drospirenone and 17-ß-estradiol, in postmenopausal women with hypertension' HYPERTENSION vol. 48, 26 June 2006, pages 246 - 253

## Description

### Field of invention

The present invention is directed to the use of treatment or Drospirenone as the sole therapeutically active agent for the prevention of hypertension.

### Background

In women, the risk of developing hypertension and/or cardiovascular diseases increases after entering the menopause, where ovulation stops (Affalo-Calderon B. HRT, women, and heart disease: what we need to know about prevention, Medscape Cardiology 6(2), 2002*).* It is thought that the treatment of post-menopausal women with estrogens would antagonise the risk of hypertension and/or cardiovascular diseases, such as hypertension. However, the risk of developing hypertension and/or cardiovascular diseases by the current forms of hormone replacement therapy has not yet been verified *(*Nelson et al. Post-menopausal hormone replacement therapy. J Am Medical Ass, August 21, 2002, vol 288, no 7, pp 872-891*).*

Hypertension has for many years been thought to be the pre-dominant factor in the development of cardiovascular diseases, but it has now been questioned to what extent anti-hypertensive agents such as ACE inhibitors prevents the development of cardiovascular diseases in women. ACE inhibitors act through maintaining electrolyte homeostasis via the renin-angiotensin-aldosterone system (RAAS) such that plasma levels of the mineralocorticoid hormone, aldosterone, are reduced due to the inhibiting of the angiotensin converting enzyme. Aldosterone is known to exert its activities through activation of mineralocorticoid receptors in the epithelia of the kidney, colon and sweat glands, whereby aldosterone promotes the retention of sodium and the excretion of potassium.

However, recently it has been shown that mineralocorticoid receptors are also present in the cardiovascular system, such as in the heart and blood vessels, as well as in the brain *(*Delayani JA: Mineralocorticoid receptor antagonists; the evolution of utility and pharmacology. Kidney INt, 2000 Apr 57(4) 1408-1411*).* Moreover, animal and human studies point to the fact that aldosterone plays a role in the pathogenesis of cardiovascular and renal diseases independent of the well-known mechanism involving activation of the angiotensin to angiotensin II *(*Stier et al, aldosterone as a mediator in cardiovascular injury. Cardiol Rev 2002, mar-Apr 10(2), 97-102*).* Thus, it is to be expected that aldosterone via its activation of the mineralocorticoid receptors in the heart, blood vessels or brain will mediate several pathophysiological actions like for example stroke, cardiac fibrosis, ventricular hypertrophy, myocardial necrosis, heart failure (congestive heart failure), sudden cardiac death and/or myocardial infarction.

Eplerenone is a steroidal drug with aldosterone receptor blocker activity. Eplerenone is reported as being effective in the treatment of aldosterone-mediated diseases such as cardiovascular diseases *(*Martin and Krum; Eplerenone. Current opinion in investigational drugs, 2001, 2(4), 521-525*).* Eplerenone has a selective binding to the mineralocorticoid receptor in that other steroidal receptors are not affected, such as the estrogenic and progestational receptors. Eplerenone has been shown to provide cardioprotection in both women and men *(*Hamedii and Chadow: The promise of selective aldosterone receptor antagonist for the treatment of hypertension and congestive heart failure. Curr Hypertens Rep, 2000, Aug 2(4), 378-383*,* Delayani et al: Eplerenone; a selective aldosterone receptor antagonist (SARA), cardiovascular Drug Rev 2001 FAII, 19(3) 185-200*).*

Another drug substance is Spironolactone (17-hydroxy-7-alpha-mercapta-3-oxo-17-alpha-pregn-4-ene-21-carboxylic acid gamma-lactone acetate), which has been shown to decrease the morbidity and mortality among patients with severe heart failure who were already receiving ACE inhibitor therapy. Thus, an additive effect was observed *(*Pitt et al; the effect of Spironolactone on morbidity and mortality in patients with severe heart failure. New Engl. J Med, vol 341, no 10, pp 709717, 1999*).*

Aldosterone antagonists have been used in the treatment of aldosterone mediated pathogenic effects such as hypertension and/or cardiovascular diseases in a subject that has salt sensitivity or an elevated dietary sodium intake or both by administering one or more aldosterone antagonists such as Spironolactone (WO 01/95892).

WO 02/09759 relates to the treatment of inflammation-related cardiovascular disorders such as aetherosclerosis in a subject by administering an aldosterone antagonist and a cyclooxygenase-2 inhibitor. The aldosterone antagonist being a spirolactone-type compound (Spironolactone) and an epoxy-steroidal aldosterone antagonist.

WO 01/34132 relates to the treatment, inhibiting or preventing pathogenic change resulting from vascular injury in a human subject by administering an aldosterone antagonist.

WO 95/15166 relates to the treatment with an aldosterone antagonist such as Spironolactone and epoxymexrenone for inhibiting myocardial fibrosis in that the dosage used may not disrupt a patient's normal electrolyte and water-retention balance.

Drospirenone (DRSP), a 17α-spirolactone is an analogue to Spironolactone. Drospirenone has unlike other known progestins biochemical and pharmacological profiles similar to endogenous progesterone, especially with regard to the anti-mineralocorticoid activity in the epithelia of the kidney, colon and sweat glands and the anti-androgenic activity *(*Krattenmacher R; Drospirenone: pharmacology and pharmacokinetics of a unique progestogen. Contraception 2000, Jul, 62(1), 29-38*;* Norman P, Drospirenone. Drugs of the future 2000, 25(12, 1247-1256*).* Therefore, Drospirenone is thought to mediate the natural anti-aldosterone and vasoactive properties of endogenous progesterone in women. It is known that Drospirenone binds to mineralocorticoid receptors in competition with aldosterone so as to provide a strong anti-mineralocorticoid activity. Drospirenone is about 8 times as potent as Spironolactone *(*Pollow et al; dihydrospirorenone, a novel synthetic progestagen, characterisation of binding to different receptor proteins. Contraception, 1992, 46, 561-574*).*

Drospirenone Is known from the patent DE 19633685 and the patent application WO 98067838, both relate to a process for producing Drospirenone, 6β,7β;15β;16β-dimethylene-3-oxo-17α-preg-4-ene-21,17-carbolactone.

Plasma aldosterone levels may be affected by administering Drospirenone: In a study implying administration of Drospirenone alone or Drospirenone in combination with ethinylestradiol demonstrated that the plasma aldosterone levels increased in both groups of therapy. *(*Oelkers et al: Effect of an oral contraceptive containing Drospirenone in the renin-angiotensin-aldosterone system In healthy female volunteers, Gynecol endocrinol 2000, 14, 204-213*).*

Furthermore, it has been shown that Drospirenone in combination with 17β-estradiol further reduces hypertension in postmenopausal women that suffer from hypertension and is in therapy with an ACE inhibitor, Enalapril *(*Preston et al, Additive effect of drospirenone/17β-estradiol in hypertensive postmenopausal women receiving Enalapril (Am J Hypertension, 2002, 15 (816-822*).*

Drospirenone is further known from its use in contraception (WO 01/15701) and for managing HRT in post-menopausal women (WO 01/52857).

Finally, subcutaneous administration of Drospirenone (2 and 10 mg, respectively) in male spontaneously hypertensive rats has been described *(*Berger et al; Influence of different progestogens on blood pressure of non-anaesthetized male spontaneously hypertensive rats. Contraception, 1992, 46, 83-97*).*

### Summary of the invention

It is currently considered problematic that the hormone replacement therapy used in the clinic today does not reduce the risk of hypertension and/or cardiovascular diseases in post-menopausal women. For many years it has been the theory that estrogen replacement therapy would reduce the occurrence of cardiovascular diseases in post-menopausal women. However, it has been found that endogenous progesterone may protect a woman from developing cardiovascular diseases because of the antagonising effect of aldosterone, such as the antagonism of aldosterone on mineralocorticoid receptors In the heart, blood vessels and brain. Endogenous levels of progesterone may begin to become deficient when women enters the age of forties or in the period of peri-menopause. After entering meno-pause, the endogenous levels of progesterone are absent.

Accordingly, it has been found that Drospirenone, a drug substance with analogous progestational as well as anti-mineralocorticoid activity as progesterone, antagonises the action of aldosterone on mineralocorticoid receptors in the heart, blood vessels and brain, resulting in the potential prevention of cardiovascular diseases in women. This observation has great significance to women with the risk of developing cardiovascular diseases, such as women with deficient levels of endogenous progesterone or women with a history of cardiovascular diseases in their family. Advantageously, women can be offered cardioprotective treatment simultaneously with the treatment of symptoms and diseases associated with deficient levels of progesterone, such as irregular bleeding, abnormal bleeding, endometrioses and symptoms on peri-menopause.

Thus, the present invention relates to the use of Drospirenone for the preparation of a solid dosage unit comprising Drospirenone as the sole therapeutically active agent for the treatment or prevention of hypertension, wherein said solid dosage unit contains a dose of 2, 2.5 or 3 mg Drospirenone.

### Detailed description

As stated, the invention lies in part in the hitherto unknown cardioprotective effect in women when being in therapy with Drospirenone. Drospirenone is a drug substance having aldosterone antagonising activity due to binding to mineralocorticoid receptors in competition with aldosterone. However, Drospirenone does also have progestational activity.

It is thought that the cardioprotective effect results from the anti-aldosterone activity of Drospirenone on mineralocorticoid receptors in the heart, the vascular system and brain.

As used herein, the following phrases and terms are defined:

The term "preventing" includes either prevention of the onset of a clinical manifestation of a symptom, disease or disorder altogether or preventing the onset of a preclinically evident stage of a disease or disorder in a woman. For example, the prevention of hypertension may be determined as the reduction in diastolic and/or systolic blood pressure.

Preferably, the therapeutic uses of the present invention reduce the risk of hypertension by at least 50%, such as at least 60, 70, 80 or 90%.

A number of animal' models for the investigation of the hypertension effect of a therapeutically active agent are available. Experiments can be carried out in rodents that are made hypertensive using surgical alterations, or in spontaneously hypertensive rodent treated with a stroke-prone substance.

For example, arterial hypertension can be induced in rodents using different techniques:
a) renovascular hypertension induced by surgically placing a constricting band around the right renal artery, to induce unilateral renal ischemia.
b) infrarenal banding to mechanically constrict blood flow through the aorta below the junction where the renal arteries branch off. (elevated blood pressure in the kidneys).
c) aldosterone infused directly into the rodents at a fixed rate via an implanted pump.

The spontaneously hypertensive rodents of the stroke-prone substrate is characterised by an increased development of severe hypertension, cerebrovascular lesions, and malignant nephrosclerosis.

During the treatment period, the rodents are monitored for hypertension, and plasma aldosterone, systolic and diastolic blood pressure, left ventricular and diastolic pressure, left ventricular dP/dt, body weight, and heart rate.

At the end of the treatment period, the rodents are sacrificed and compared between the different groups of treated with either an active agent or placebo, by examination of the heart, microscopic evaluation of the cerebrovascular damages and/or histopathologic analysis of the kidneys.

Other models of investigating hypertension effects are mentioned in Delyani et al; effect of a selective aldosterone receptor antagonist in myocardial infarction. Am J Physiol Heart Circ Physiol, vol 50, H647-H654, 2001*.*

The term "Drospirenone" is intended to include any geometric isomer of Drospirenone.

As stated, Drospirenone is the only therapeutically active agent included in the dosage unit. In the present context, the term "sole therapeutically active agent" is denoted to mean that substantially no other drug substances is included in the dosage unit. However, some drug substances can be allowed as long as they are not regarded as therapeutically relevant for the treatment of hypertension or are present in a dose insufficient to prevent or treat hypertension . Moreover, other hormonal agents such as estrogens are excluded from the composition.

The composition is intended for being administered to a woman so as to treat or prevent the development of hypertension. The administered dose is, 2, 2.5, or 3 mg dependent on the woman and the intention of preventing or treating hypertension

Unlike earlier publications, it is found that prevention of hypertension in a woman can sufficiently be achieved upon administering the Dropirenone as the sole therapeutically active agent. That is to say that co-administering of Drospirenone with anti-hypertensive drugs or estrogens is not critical for achieving sufficient prevention of hypertension and/or cardiovascular diseases in a woman.

The therapeutic uses the invention are directed to women having the need or desire of being protected from hypertension. In general, the therapy and medicaments of the invention are directed to a woman above a specified age for reducing the risk of hypertension. Therefore, in some preferable embodiments, the therapeutic uses are applied to a woman above a critical age, for example a woman who may have no clinical symptoms of hypertension, the only criterion being that she is above a critical age. The critical age will vary from population to population according to the incidence of cardiovascular disease or according to other factors such as diet or smoking. The critical age can be determined simply by determining the age above which 80%, such as 85%, 90% or 95% of the deaths from heart failure or cardiac infarct occur. Typically the characteristic age is above 35, such as above 40, 45 and 47, and may range from about the age from about 40 to 65.

In some embodiments of the invention, the therapeutic uses of the invention are intended to healthy women irrespective of their age, e.g. women with a systolic blood pressure less than 140 mm Hg such as less than 130 mm Hg or 120 mm Hg or a diastolic blood pressure less than 105 mm Hg, such as less than 95 mm Hg, 85mm Hg or 75 mm Hg or combinations of said systolic blood pressure and diastolic blood pressure. An alternative characteristic of a healthy woman relates to the body mass Index. Thus, in some embodiments of the invention, the woman has a body mass index in the range from about 16 to 35 in that the body mass index in older woman may be in the higher end of that range without being denoted obesive. Normally, to ensure low risk of hypertension , the body-mass index should be lower than 35. Therefore, the body-mass index of a woman is preferably in the range from about 18 to 32, even more preferably in the range from about 18 to 29, most preferably in the range from about 19 to 28, such as most preferably in the range from about 20 to 27.

Alternatively, the therapy of the invention is used in women with an estimated risk of hypertension above a specified level,
wherein the risk is determined by measurement of risk factors used in conjunction with a person's age and sex. Thus, in one embodiment, the woman is susceptible to an aldosterone-mediated disease, such as hypertension . Thus, in some embodiments the woman can be hypertensive.

Moreover, a woman susceptible to hypertension may be characterised by having deficient endogenous levels of progesterone, such as a woman in peri-menopause or in post-menopause or a woman with irregular or abnormal bleeding, or such as a pregnant woman in risk of miscarriage. Thus, in some embodiments of the invention, the therapeutic methods or medicaments of the invention may be used to women having symptoms, diseases or disorders associated with deficient endogenous levels of progesterone, or to women in need of progesterone replacement therapy, such as a woman having irregular bleeding, abnormal bleeding, endometrioses and/or lack of ovulation.

Typically, the therapeutic uses may also be applied to a healthy woman but nevertheless in the risk of developing hypertension . The woman may previously have been diagnosed as having had the clinical symptoms of hypertension, irrespective of age or the values of risk factors. Therefore, in some embodiments of the invention, the woman having risk or even Increased risk of hypertension is a woman with a systolic blood pressure greater than 130 mm Hg or a diastolic blood pressure greater than 85 mm Hg or both. Furthermore, the woman in risk may also be characterised by having activities ratio of plasma aldosterone (ng/dL) to plasma renin (ng/mL/hr) greater than about 30. Women with risk factors can promote the development of arteriosclerosis. Such risk factors are smoking, overweight, stress, genetic disposition, high blood pressure, disorders of lipid metabolism, lack of exercise and/or diabetes.

In particular embodiments of the invention, the therapeutic uses are preferably directed to a peri-menopausal woman.

The term "peri-menopause" characterises the period immediately before and after the onset of menopause, and averages 4 years. Furthermore, the peri-menopause is characterised by abnormal and irregular bleeding. The peri-menopausal phase begins with the onset of climacteric symptoms when the cycle becomes irregular and ends one year after menopause. The end of peri-menopausal phase can be identified after a protracted period of time without bleeding.

That is to say that the peri-menopause is the period before entering menopause. Thus, in interesting embodiments of the invention the woman is in the age from about 40 to 55. The suitable age may also be from about 45 to 53, about 45 to 52, 46 to 52, 48 to 52 or from about 47 to 51 dependent on when the individual women experiences irregular bleeding or other symptoms on deficient endogenous levels of progestogen. However, the uses of the invention may not be limited to women of that age. Some women enter the menopause in a much younger age such as for example when they are in theirs thirties due to hypogonadal activities. Other women will enter the menopause upon hysterectomy.

As mentioned, low endogenous levels of progesterone are an indication of peri-menopause in a woman. However, post-menopausal women does also have low endogenous levels of progesterone or even lack of progesterone for which reason post-menopausal women may benefit from the additional prevention of development of hypertension upon administering Drospirenone according to the invention . As mentioned, the therapeutic methods and uses do not necessarily imply that the post-menopausal women are in estrogen replacement therapy.

The term "menopause" characterises the time in a woman's life when the ovaries stop producing estrogen. Menopause is usually recognised by the cessation of menstrual periods. Other symptoms of menopause include flashes, mood changes, and difficulty in sleeping and vaginal dryness. In the present context, the term menopause is to be understood as the last natural (ovary -induced) menstruation. If a woman is not menstruating because she has had a hysterectomy or endometrial ablation, other symptoms of menopause often alert her that menopause is starting. The average age of the onset of menopause is 51 years, and it most commonly occurs from age 47 to 53. The term "post-menopause" is the phase that begins at menopause and continues until death.

Accordingly, in some embodiments of the invention, the woman is a post-menopausal woman, e.g. a woman with deficient levels of endogenous estrogen.

The therapeutic uses of the invention do not comprise concurrent administration of an estrogen i.e. an estrogen is excluded.

The Drospirenone may be delivered daily for a number of days within each cycle of 21 to 35 days, preferably within each cycle of 28 days. Cycle is intended to mean a period of time that is repeated for every 21 to 35 days. In some embodiments the delivery of the Drospirenone may be for at least 5 days up to 35 days.

In other embodiments the Drospirenone is delivered for at least 10 days, such as 12 days, such as 15 days, such as 20 days, such as 21, 22, 23, 24, 25, 26, 27, or 28 days within each cycle of 21 to 35 days, preferably 28 days. In some embodiments the delivery is in interrupted manner, which means that the Drospirenone is delivered for a period of time, which is followed by a period with no Drospirenone delivered, which is then followed by a period with delivery of the Drospirenone. Also, it means that no Drospirenone is delivered for a period of time, followed by delivery of Drospirenone, followed by no delivery of Drospirenone. This cycling of delivery of Drospirenone may be repeated 1 to 10 times within a cycle of 21 to 35 days. That is to say that each delivery of Drospirenone may for example be conducted for 1, 3, 7, 14 or 21 days. The length of the period with delivery of Drospirenone may be similar, minor or greater than the period with no delivery. In some embodiments, the Drospirenone is administered sequentially, which relates to an interrupted manner.

In other embodiments, the Drospirenone is administered continuously within a treatment cycle of 21 to 35 days.

The Drospirenone dose may vary throughout the period of 21 to 35 days or it may be the same. Thus, in some embodiments, the dose delivered varies throughout the period of 21 to 35 days. For example, the dose may be lower in the first half, such as within the first 10 days, of the period of 21 to 35 days than in the second half, such as the last 10 days, of the period of 21 to 35 days. Conversely, it may also be suitable to use a higher dose in the first half, such as within the first 10 days, of the period of 21 to 35 days than in the second half, such as the last 10 days of the period of 21 to 35 days.

The composition to be used according to the invention may be formulated in any suitable manner. In suitable embodiments of the invention, the composition is formulated , as a solid dosage unit for oral administration.

The solid dosage units may be selected from the group consisting of uncoated tablets, modified-release tablets, gastro-resistant tablets, orodispersible tablets, effervescent tablets, chewable tablets, soft capsules, hard capsules, modified-release capsules, gastro-resistant capsules, uncoated granules, effervescent granules, coated granules, gastro-resistant granules, modified -release granules, and powders for oral administration.

Moreover, an interesting embodiment of the invention comprises a dosage unit wherein the drospirenone is in micronized form or in the form of a cyclodextrin inclusion complex.

The dosage unit of the present invention comprises carriers or excipients, which may act to promote dissolution of both active substances. Examples of such carriers and excipients include substances that are readily soluble in water such as cellulose derivatives, carboxymethyl cellulose, hydroxypropyl cellulose, hydroxypropyl methyl cellulose, gelled starch, gelatin or polyvinylpyrrolidone. In particular, it is anticipated that polyvinylpyrrolidone might be particularly helpful to promote dissolution.

The term "pharmaceutically acceptable carriers and excipients" is intended to mean substances, which are substantially harmless to the individual to which the dosage unit will be administered. Such an excipient normally fulfils the requirements given by the national drug agencies. Official pharmacopeias such as the British Pharmacopeia, the United States of America Pharmacopeia and the European Pharmacopeia set standards for well-known pharmaceutically acceptable excipients.

Suitable pharmaceutically acceptable excipients according to the invention include all kinds that may be used for solid dosage units.

### Example 1

A composition comprising Drospirenone may be manufactured in the following manner:

**Tablet cores of the following composition**

| | |
|---|---|
| Drospirenone (preferably micronised) | 3.00 mg |
| lactose monohydrate | 45.2 mg |
| corn starch | 14.40 mg |
| modified starch | 9.60 mg |
| polyvinylpyrrolidone 25,000 | 4.00 mg |
| magnesium stearate | 0.80 mg |

are prepared by charging a fluidised bed granulator with corn starch, modified starch, drospirenone, lactose monohydrate and activating the fluidised bed. An aqueous solution of polyvinylpyrrolidone 25,000 in purified water is sprayed continuously onto the fluidised bed while drying by heating the air stream of the fluidised bed. At the end of the process magnesium stearate is sucked into the granulator and mixed with the granules by maintaining the fluidised bed. The resulting granulate is pressed into tablet cores by compression using a rotary tablet press.

## Claims

1. Use of drospirenone for the preparation of a solid dosage unit comprising drospirenone as the sole therapeutically active agent for the treatment or prevention of hypertension, wherein said solid dosage unit contains a dose of 2, 2.5 or 3 mg drospirenone.

2. Use according to claim 1, wherein said solid dosage unit is for the treatment or prevention of hypertension in a woman.

3. Use according to claim 2, wherein said woman Is a peri-menopausal woman.

4. Use according to claim 2, wherein said woman is a post-menopausal woman.

5. Use according to any of the preceding claims, wherein said drospirenone is micronized.

## Patentansprüche

1. Verwendung von Drospirenon zur Herstellung einer festen, Drospirenon als einzigen therapeutischen Wirkstoff enthaltenden Dosierungseinheit zur Behandlung oder Prävention von Bluthochdruck, wobei die feste Dosierungseinheit eine Dosis von 2, 2,5 oder 3 mg Drospirenon enthält.

2. Verwendung nach Anspruch 1, wobei die feste Dosierungseinheit für die Behandlung oder Prävention von Bluthochdruck bei einer Frau bestimmt ist.

3. Verwendung nach Anspruch 2, wobei es sich bei der Frau um eine perimenopausale Frau handelt.

4. Verwendung nach Anspruch 2, wobei es sich bei der Frau um eine postmenopausale Frau handelt.

5. Verwendung nach einem der vorhergehenden Ansprüche, wobei das Drospirenon mikronisiert ist.

## Revendications

1. Utilisation de la drospirénone pour la préparation d'une unité de dosage solide comprenant la drospirénone en tant que seul agent thérapeutiquement actif destiné au traitement ou à la prévention de l'hypertension, **caractérisée en ce que** ladite unité de dosage solide contient une dose de 2, 2,5 ou 3 mg de drospirénone.

2. Utilisation selon la revendication 1, **caractérisée en ce que** ladite unité de dosage solide est destinée au traitement ou à la prévention de l'hypertension chez une femme.

3. Utilisation selon la revendication 2, **caractérisée en ce que** ladite femme est une femme péri-ménopausée.

4. Utilisation selon la revendication 2, **caractérisée en ce que** ladite femme est une femme post-ménopausée.

5. Utilisation selon l'une quelconque des revendications précédentes, **caractérisée en ce que** ladite drospirénone est micronisée.
